# EUROPEAN PATENT SPECIFICATION

(11) **EP 3 801 457 B1**
(45) Date of publication and mention of the grant of the patent: **20.05.2026**
(21) Application number: 19732190.4
(22) Date of filing: 04.06.2019
(51) Int. Cl.: A61K 9/00, A61K 31/167, A61K 31/573, A61K 45/06, A61P 11/00, A61P 11/02, A61P 11/06, A61P 11/08

(54) **STABLE PHARMACEUTICAL COMPOSITIONS FOR PRESSURIZED METERED DOSE INHALERS**
STABILE PHARMAZEUTISCHE ZUSAMMENSETZUNGEN FÜR DRUCKDOSIERINHALATOREN
COMPOSITIONS PHARMACEUTIQUES STABLES POUR INHALATEURS DOSEURS SOUS PRESSION

(30) Priority: 04.06.2018 US 201862680173 P
(43) Date of publication of application: 14.04.2021
(73) Proprietor: Lupin Inc., Coral Springs, Florida 33065 (US)
(72) Inventor: DALVI, Mukul, Coral Springs, Florida 33065 (US); GUPTA, Abhishek, Coral Springs, Florida 33065 (US); COLOMBANI, Agnes, Coral Springs, Florida 33065 (US)
(74) Representative: J A Kemp LLP
(86) International application number: PCT/US2019/035362
(87) International publication number: WO 2019/236559

(56) References cited:
- EP-A1- 1 982 709
- EP-A2- 1 787 639
- EP-A2- 2 486 914
- WO-A1-2004/019985
- WO-A1-2012/049444
- WO-A1-2017/093755
- WO-A2-2007/020204
- WO-A2-2012/007729
- WO-A2-2012/110770
- US-A1- 2011 150 782
- CHIESI LIMITED: "Fostair 100/6 micrograms per actuation pressurised inhalation solution", MEDICINES.ORG.UK, 15 May 2017 (2017-05-15), XP055658129, Retrieved from the Internet <URL:https://www.medicines.org.uk/emc/files/pil.6318.pdf> [retrieved on 20200115]

## Description

### CROSS REFERENCE TO RELATED APPLICATIONS

This application is being filed on 4 June 2019, as a PCT International patent application, and claims priority to U.S. Provisional Patent Application No. 62/680,173, filed June 4, 2018.

### FIELD OF THE INVENTION

The present invention provides a stable pharmaceutical composition comprising a β2 agonist, or a combination of a β2 agonist and an inhaled corticosteroid and/or a long acting muscarinic antagonist, a propellant, a co-solvent, an organic acid(s) and optionally water.

### BACKGROUND OF THE INVENTION

Metered dose inhalers (MDIs) are important and well known devices used to deliver appropriate therapy for a growing number of respiratory diseases. MDIs are a convenient, inexpensive delivery system and are widely used. Today, more than 20 different metered dose inhalers based products are on the market to deliver active pharmaceutical ingredients (APIs) for local and/or systemic therapy.

MDIs when actuated create propellant droplets containing the pharmaceutical product for delivery to the respiratory tract as an aerosol. Formulations for aerosol administration via MDIs can be solutions or suspensions. Solution formulations offer the advantage of being homogeneous, with the active ingredient and excipients completely dissolved in the propellant vehicle or its mixture with suitable co-solvents such as ethanol. Solution formulations also obviate physical stability problems of micronized particles associated with suspension formulations so assuring more consistent and uniform dosage administration. Since solution formulations provide smaller mass median aerodynamic diameters (MMADs) they facilitate delivery to the entire lung including the alveolar regions. The mass median aerodynamic diameter is generally measured with a cascade impactor.

However, a significant challenge associated with solution formulations is the chemical stability of the active ingredients. The loss of active substance content should be minimized during the shelf-life and the appearance of degradative product should be diminished so that the medicament can be used with its effectiveness and safety. It means, the physical and chemical stability as well as the maintenance of the quality parameters during the shelf-life of the aerosol are of essential importance for its practical medical use.

There has been a common challenge in preparing solution formulations containing β2-agonist because they are often chemically unstable especially in the presence of co-solvents such as ethanol which are necessary to formulate the solutions. Thus, the investigations have been directed vastly to avoid the degradation of β2-agonist.

EP 1787 639 B1 discloses an aerosol solution composition which comprises a β2-agonist drug of the phenylalkylamino class bearing a functional group sensitive to oxidative and/or hydrolytic reaction in a solution of a liquefied HFA propellant, a co-solvent selected from pharmaceutically acceptable alcohols, wherein the apparent pH of the solution is comprised between 2.5 and 5.0 by addition of small amounts of a mineral acid selected from hydrochloric, nitric and phosphoric acid, wherein the active ingredient is formoterol as β2-agonist drug or a salt thereof in combination with a steroid selected from beclometasone dipropionate, fluticasone propionate, budesonide and its 22R-epimer or an anticholinergic atropine like derivative selected from ipratropium bromide, oxitropium bromide and tiotropium bromide.

EP 2 223 682 B1 discloses an aerosol composition which consists of an active ingredient formoterol fumarate in combination with beclometasone diproprionate in a solution of a liquefied HFA 134a propellant and 12% w/w ethanol as a co-solvent, and hydrochloric acid in an amount such that the solution has an apparent pH between 3.0 and 3.5.

EP 2 010 190 discloses a pressurized solution formulation for a metered dose inhaler comprising formoterol fumarate in combination with beclometasone dipropionate as active substances dissolved in a mixture consisting of HFA134a propellant and an amount of ethanol of 12% w/w as a co-solvent and 0.024% w/w hydrochloric acid (1M) for use in the prevention and/or treatment of a severe broncho-pulmonary disease selected from severe persistent asthma or severe or very severe chronic obstructive pulmonary disease (COPD), wherein, upon actuation of said inhaler, 50 µl of said solution comprising formoterol fumarate at a dose per actuation of 6 µg and beclometasone dipropionate at a dose of 100 µg per actuation are metered for delivery.

EP 1 660 035 discloses a pharmaceutical compositions are propellant free solutions intended for nebulization.

US 2006/0140873 discloses a composition for use in a metered dose inhaler (MDI), the composition comprising predetermined amounts of an active pharmaceutical ingredient (API) insoluble in the composition, a propellant comprising a hydrofluoroalkane (HFA), and a pharmaceutically acceptable non-aminated C1-6 organic acid that increases post-shaking suspension time of the API in the composition to at least 30 seconds to provide uniform dosing of the API from the inhaler over at least 30 seconds post-shaking.

EP 2 486 914 A2 discloses pharmaceutically stable pressurized metered dose inhaler composition comprising two active pharmaceutical ingredients together, out of which one active agent is suspended, and other active agent is dissolved in a formulation along with other excipient such as cosolvent, dissolved halide, propellant, water, organic acid and unsaturated suspending agent.

WO 2007/020204 A2 discloses the aerosol formulations of acid addition salbutamol salt preferably salbutamol hydrochloride or salbutamol citrate together with HFC propellant, co-solvent and either an inorganic or an organic acid.

WO 2012/110770 A2 discloses pharmaceutical compositions for inhalation comprising glycopyrrolate in combination with a beta2-agonist selected from indacaterol, formoterol, vilanterol, carmoterol, olodaterol, and optionally, one or more pharmaceutically acceptable excipients to the use for the prevention and/or treatment of respiratory, inflammatory or obstructive airway disease.

WO 2012/049444 A1 relates to pharmaceutical composition comprising a beta2-agonist selected from indacaterol and formoterol in combination with a corticosteroid selected from fluticasone and ciclesonide, along with one or more pharmaceutically acceptable excipients.

WO 2012/007729 A2 discloses the pharmaceutical composition for inhalation comprising (+) budesonide with one or more bronchodilators.

WO 2004/019985 A1 discloses pharmaceutical compositions comprising specific anticholinergic agents, β-2 agonists and corticosteroids for use in the treatment of asthma and related disorders.

WO 2017/093755 A1 discloses pharmaceutical composition comprising at least one mometasone compound selected from mometasone and the pharmaceutically acceptable derivatives thereof; and a propellant component comprising 1,1-difluoroethane (R-152a).

EP 1 787 639 A2 discloses stable pharmaceutical aerosol formulation composition comprising a β2-agonist drug selected from formoterol, salmeterol, salbutamol, TA-2005 and salts in combination with a steroid such as beclomethasone dipropionate, fluticasone propionate, budesonide and its 22R-epimer or an anticholinergic atropine-like derivative such as ipratropium bromide, oxitropium bromide, tiotropium bromide along with a solution of a liquefied HFA propellant, a co-solvent, wherein the pH of the solution is comprised between 2.5 and 5.0 by addition of small amounts of a mineral acid such as hydrochloric, nitric or phosphoric acid.

US 2011/0150782 A1 discloses stable pharmaceutical composition, comprising glycopyrronium bromide, formoterol or a salt thereof dissolved in an HFA propellant, co-solvent, and solution is stabilized by the addition of an acid in an amount equivalent to 0.1 to 0.3 µg/µl of 1M HCl.

EP 1 982 709 A1 discloses the use of compositions comprising fixed dose combination of formoterol, a pharmaceutically acceptable salt or solvate thereof or a solvate of such a salt and beclomethasone dipropionate for prevention or treatment of an acute condition of asthma, intermittent asthma and/or episodes in chronic asthma. A pressurized metered dose inhaler solution formulation comprises formoterol fumarate, beclomethasone dipropionate, ethanol 12% w/w as cosolvent, hydrochloric acid (1M) 0,024% w/w as stabilizing agent and HFA 134a to 100% as the propellant.

Patient leaflet of "Fostair 100/6 micrograms per actuation pressurised inhalation solution", Chiesi Limited discloses pharmaceutically active and inactive ingredients of Fostair, wherein active ingredients are beclometasone dipropionate, formoterol fumarate dihydrate and inactive ingredients are ethanol anhydrous, hydrochloric acid and the CFC-free propellant -norflurane (HFA-134a).

The present disclosure discloses a pharmaceutical composition comprising to be used with pressurized metered dose inhalers and comprises of one or a combination of active ingredients, specifically a β2 agonist or a combination of a β2 agonist and an inhaled corticosteroid and/or a long acting muscarinic antagonist, a propellant, a co-solvent, an organic acid(s) and optionally water.

### SUMMARY OF THE INVENTION

The references to the methods of treatment by therapy of this description are to be interpreted as references to compounds, pharmaceutical compositions and medicaments of the present invention for use in those methods.

The present invention is defined by the appended claims.

According to one aspect of the present disclosure there is provided a pharmaceutical composition comprising β2-agonist, propellant, a co-solvent and an organic acid(s).

According to another aspect of the present disclosure there is provided a pharmaceutical composition comprising β2-agonist, propellant, a co-solvent, an organic acid(s) and water.

According to one aspect of the present disclosure there is provided a pharmaceutical composition comprising long acting β2-agonist, propellant, a co-solvent and an organic acid(s).

According to one aspect of the present disclosure there is provided a pharmaceutical composition comprising short acting β2-agonist, propellant, a co-solvent and an organic acid(s).

According to one aspect of the present disclosure there is provided a pharmaceutical composition comprising long acting β2-agonist, propellant, a co-solvent, an organic acid(s) and water.

According to one aspect of the present disclosure there is provided a pharmaceutical composition comprising short acting β2-agonist. propellant, a co-solvent, an organic acid(s) and water.

According to one aspect of the present disclosure there is provided a pharmaceutical composition comprising formoterol fumarate, HFA134a, maleic acid, and ethanol.

According to one aspect of the present disclosure there is provided a pharmaceutical composition comprising formoterol fumarate, HFA227ea, maleic acid, and ethanol.

According to one aspect of the present disclosure there is provided a pharmaceutical composition comprising formoterol fumarate, HFA134a, maleic acid, ethanol and water.

According to one aspect of the present disclosure there is provided a pharmaceutical composition comprising formoterol fumarate, HFA227ea, maleic acid, ethanol and water.

According to one aspect of the present disclosure there is provided a pharmaceutical composition comprising β2-agonist, corticosteroid, propellant, a co-solvent and an organic acid(s).

According to one aspect of the present disclosure there is provided a pharmaceutical composition comprising β2-agonist, corticosteroid, propellant, a co-solvent, an organic acid(s) and water.

According to one aspect of the present invention there is provided a pharmaceutical composition comprising formoterol fumarate, beclometasone dipropionate, HFA134a, ethanol and maleic acid.

According to one aspect of the present invention there is provided a pharmaceutical composition comprising formoterol fumarate, beclometasone dipropionate, HFA227ea, ethanol and maleic acid.

According to one aspect of the present invention there is provided a pharmaceutical composition comprising formoterol fumarate, beclometasone dipropionate, HFA134a, ethanol, maleic acid and water.

According to one aspect of the present invention there is provided a pharmaceutical composition comprising formoterol fumarate, beclometasone dipropionate, HFA227ea, ethanol, maleic acid and water.

According to one aspect of the present disclosure there is provided a pharmaceutical composition comprising β2-agonist, anticholinergic drug, propellant, a co-solvent and an organic acid(s).

According to one aspect of the present disclosure there is provided a pharmaceutical composition comprising β2-agonist, anticholinergic drug, propellant, a co-solvent, an organic acid(s) and water.

According to one aspect of the present disclosure there is provided a pharmaceutical composition comprising long acting β2-agonist, corticosteroid, anticholinergic drug, propellant, a co-solvent and an organic acid(s).

According to one aspect of the present disclosure there is provided a pharmaceutical composition comprising long acting β2-agonist, corticosteroid, anticholinergic drug, propellant, a co-solvent, an organic acid(s) and water.

According to one aspect of the present invention there is provided a pharmaceutical composition comprising formoterol fumarate, beclometasone dipropionate, Glycopyrrolate, HFA134a, ethanol, maleic acid and water.

According to one aspect of the present invention there is provided a pharmaceutical composition comprising formoterol fumarate, beclometasone dipropionate, Glycopyrrolate, HFA227ea, ethanol, maleic acid and water.

### DETAILED DESCRIPTION

The present disclosure relates to a pharmaceutical composition comprising β2-agonist, a propellant, a co-solvent, an organic acid(s) and optionally water. The pharmaceutical composition is a solution or a suspension, preferably a solution.

The aerosol medicinal products are very important as pharmaceutical dosage forms for drug administration by pulmonary route. The physical and chemical stability as well as the maintenance of the quality parameters, during the shelf-life of the aerosol, are of essential importance for its practical medical use.

Solution compositions eliminate the typical concerns around physical stability of micronized particles required for lung delivery and ensure that a consistent particle size is achieved through the shelf-life of the product. Solution compositions also provide smaller Mass Median Aerodynamic Diameter (MMAD) and therefore facilitate delivery to the entire lung including the alveolar regions.

There has been a common challenge in preparing solution compositions containing β2-agonist as one of the actives because they are often chemically unstable especially in the presence of co-solvents such as ethanol which are necessary to formulate the solutions. Thus, the investigations have been directed vastly to avoid the degradation of β2-agonist.

The preparation of stable solution formulations is even more vital when bronchodilator β2-agonists having benzylic hydroxyl group, like formoterol, albuterol, and others, may suffer from inherent chemical stability due to their susceptibility to substitution by nucleophilic species in the formulation via SN1 or SN2 reactions. Ethanol can satisfy the role of a nucleophile and cause degradation of β2-agonist having benzylic hydroxyl group.

Surprisingly, it has been found that maleic acid, which have much weaker proton donating ability, can stabilize β2-agonist in solution formulations containing co-solvent such as ethanol for delivery using pressurized metered dose inhalers.

The present disclosure provides a pharmaceutical composition comprising β2-agonist, a propellant, a co-solvent, an organic acid(s) and optionally water.

When water is present in the pharmaceutical compositions, it may present up to 5% by wt, more preferably up to 3% by wt, and the most preferably up to 1 % by wt.

In accordance with the invention, β2-agonist is present in the composition in an amount between approximately 0.005 % by weight and 1 % by weight.

In accordance with the invention, the propellant of the invention includes, HFA 134a (1,1,1,2-tetrafluoroethane), HFA 227ea (1,1,1,2,3,3,3 -heptafluoropropane), HFA 152a (1,1-difluoroethane) or mixtures thereof.

The co-solvent of the invention includes, dichloromethane, chloroform, ethylacetate, N-methyl pyrrolidone, benzylalcohol, isopropylacetate, acetonitrile, tetrahydrofuran, isopropanol, methanol, ethanol or mixtures thereof. Preferably the co solvent is ethanol; and it is present in an amount between approximately 1 % by weight and 40% by weight, more preferably between 4 % by weight and 20 % by weight.

In accordance with the disclosure, "the organic acid" term refers to any organic compound with acidic properties. An organic acid is different from an inorganic acid (or mineral acid) that is derived from one or more inorganic compounds. Organic acids tend to have weaker proton donating ability than inorganic acids. The organic acid of the invention includes, maleic acid, fumaric acid, citric acid, acetic acid, xinafoic acid, oxalic acid, lactic acid, 2-methyl propionic acid, malic acid, butanoic acid, tartaric acid, propionic acid, pentanoic acid, succinic acid, glycolic acid, hexanoic acid, malonic acid, glutaric acid, formic acid, adipic acid, ascorbic acid, benzoic acid, glucuronic acid or mixtures thereof. Preferably the organic acid is maleic acid; and it is present in an amount between approximately 0.001 and 1% by weight, more preferably between 0.001 % by weight and 0.10 % by weight.

The pharmaceutical composition further comprises a second active agent. The second active agent is a corticosteroid or long acting muscarinic receptor antagonist (LAMA).

The corticosteroid of the disclosure includes, beclomethasone or beclometasone dipropionate, budesonide, ciclesonide, flunisolide, fluticasone propionate, fluticasone furoate, mometasone furoate, triamcinolone acetate, or prednisolone. Preferably the corticosteroid is beclometasone or beclometasone dipropionate; and it is present in an amount between approximately 0.001 % by weight and 1% by weight, more preferably between 0.05 % by weight and 0.30 % by weight.

The long acting muscarinic receptor antagonist (LAMA) of the disclosure includes, umeclidinium, aclidinium, glycopyrronium, ipratropium, oxitropium, tiotropium or pharmaceutically acceptable salts thereof. Preferably the long acting muscarinic receptor antagonist (LAMA) is glycopyrronium, and it is present in an amount between approximately 0.001 % by weight and 1 % by weight, more preferably between 0.05 % by weight and 0.30 % by weight.

Another embodiment provides the pharmaceutical composition comprising 2-agonist, a propellant, a co-solvent, an organic acid(s) and optionally water filled in a container having part or all of its internal metallic surfaces made of stainless steel, anodised aluminium or lined with an inert organic coating.

Another embodiment provides the use of pharmaceutical composition comprising β2-agonist, a propellant, a co-solvent, an organic acid(s) and optionally water for the treatment or prophylaxis of asthma, COPD, rhinitis or as adjunct therapy for cystic fibrosis, non-cystic fibrosis bronchiectasis, lung infections or pulmonary fibrosis.

In accordance with the present invention, compositions of the present invention are less prone to hydrolytic and/or oxidative degradation. Thus, the most physically and chemically stable formulations of the invention have a significant decrease in total degradation of product. In addition to the economic advantages, the formulations in accordance with the present invention remain stable at the range of temperatures to which these medicaments are normally exposed. The person having ordinary skill in the art can also easily determine the presence of hydrolytic and/or oxidative degradation products, for example, by HPLC analysis.

The aerosol container consists of a metal canister and a measuring dosage valve having diverse plastic (typically polyester or polyamide), metal and elastomer surfaces. The use of coated containers has been found to confer an additional protection not only for the chemical stabilization of active substance but also for the absence of corrosion or other unacceptable deterioration signs of the container material by contact with the product.

The following examples are included to demonstrate particular embodiments of the invention.

### Example 1

An aerosol formulation may be prepared with the following composition:

| **Sr. No.** | **Ingredient** | **% by weight** |
|---|---|---|
| 1 | Beclometasone dipropionate | 0.172 |
| 2 | Formoterol fumarate dihydrate | 0.010 |
| 3 | Ethanol | 9.5 |
| 4 | Maleic acid | 0.003 |
| 5 | HFA134a | q.s. |

This solution formulation is filled under pressure into a canister fitted with a metering valve having a 50µl metering chamber.

### Manufacturing process 1:

1. Weighed quantity of beclometasone dipropionate along with formoterol fumarate dihydrate are dissolved in between 30-70% ethanol. Stir till a clear solution is obtained.
2. Weighed quantity of maleic acid is dissolved in the remaining ethanol. Stir the solution.
3. The solution of step 1 is mixed with solution of step 2 and charge the mixture into the batch vessel.
4. Charge part of the HFA134a propellant into the batching vessel and stir to homogenize.
5. Fill concentrate into canisters.
6. Fill balance of propellant

### Example 2

An aerosol formulation may be prepared with the following composition.

| **Sr. No.** | **Ingredient** | **°/0 by weight** |
|---|---|---|
| 1 | Beclometasone dipropionate | 0.271 |
| 2 | Formoterol fumarate dihydrate | 0.008 |
| 3 | Ethanol | 9.5 |
| 4 | Maleic acid | 0.002 |
| 5 | HFA134a | q.s. |

This solution formulation is filled under pressure into a canister fitted with a metering valve having a 63µl metering chamber. The formulation is manufactured using Manufacturing Process I described above.

### Example 3

An aerosol formulation may be prepared with the following composition.

| **Sr. No.** | **Ingredient** | **% by weight** |
|---|---|---|
| 1 | Beclometasone dipropionate | 0.172 |
| 2 | Formoterol fumarate dihydrate | 0.010 |
| 3 | Ethanol | 12 |
| 4 | Maleic acid | 0.003 |
| 5 | Water | 0.5 |
| 6 | HFA134a | q.s. |

This solution formulation is filled under pressure into a canister fitted with a metering valve having a 50µl metering chamber.

### Manufacturing process 2:

1. Weighed quantity of beclometasone dipropionate along with formoterol fumarate dihydrate are dissolved in between 30-70% ethanol. Stir till a clear solution is obtained.
2. Weighed quantity of maleic acid is dissolved in water and the remaining ethanol. Stir the solution.
3. The solution of step 1 is mixed with solution of step 2 and charge the mixture into the batch vessel.
4. Charge part of the HFA134a propellant into the batching vessel and stir to homogenize.
5. Fill concentrate into canisters.
6. Fill rest of propellant

### Example 4

An aerosol formulation may be prepared with the following composition.

| **Sr. No.** | **Ingredient** | **% by weight** |
|---|---|---|
| 1 | Beclometasone dipropionate | 0.271 |
| 2 | Formoterol fumarate dihydrate | 0.010 |
| 3 | Ethanol | 12 |
| 4 | Maleic acid | 0.002 |
| 5 | Water | 0.5 |
| 6 | HFA134a | q.s. |

This solution formulation is filled into a canister fitted with a metering valve having a 63µl metering chamber followed by the addition of the propellant.

### Manufacturing process 3:

1. Weighed quantity of beclometasone dipropionate along with formoterol fumarate dihydrate are dissolved in ethanol. Stir till a clear solution is obtained.
2. Weighed quantity of maleic acid dissolved in water and the remaining ethanol. Stir the solution.
3. The solution of step 1 are mixed with solution of step 2 and stirred to homogenize.
4. Fill concentrate into canisters.
5. Fill the required propellant into the canisters.

### Example 5 - Reference example

An aerosol formulation may be prepared with the following composition.

| **Sr. No.** | **Ingredient** | **% by weight** |
|---|---|---|
| 1 | Beclometasone dipropionate | 0.172 |
| 2 | Formoterol fumarate dihydrate | 0.010 |
| 3 | Ethanol | 16 |
| 4 | HFA134a | q.s. |

This solution formulation is filled under pressure into a canister fitted with a metering valve having a 50µl metering chamber.

### Manufacturing process 4:

1. Weighed quantity of beclometasone dipropionate along with formoterol fumarate dihydrate are dissolved in ethanol. Stir till a clear solution is obtained.
2. Charge part of the HFA134a propellant into the batching vessel and stir to homogenize.
3. Fill concentrate into canisters.
4. Fill balance of propellant.

### Example 6

A design of experiments was performed with formulations prepared with the following compositions:

| **Sr. No.** | **Ingredient** | **% by weight** |
|---|---|---|
| 1 | Beclometasone dipropionate | 0.172 |
| 2 | Formoterol fumarate dihydrate | 0.010 |
| 3 | Ethanol | 12 |
| 4 | Maleic acid | 0.0024 - 0.0033 |
| 5 | Water | 0 - 0.75 |
| 6 | HFA134a | q.s. |

Each solution formulation is filled into a canister fitted with a metering valve having a 50µl metering chamber followed by the addition of the propellant. The formulations are manufactured using Manufacturing Process 3 described above.

### Brief procedure for determination of Assay/ Total degradation product

Assay and related substances determinations are carried out using a Liquid Chromatograph (LC) by recovering the drug substances from the canisters with appropriate diluent systems composed of water and organic solvents. The test samples are run on the LC using a C18 column with a suitable organic: aqueous mobile phase. Peak identification and quantitation is carried out from the resulting chromatography.

### Assay data (Formoterol fumarate)

**Table 1**

| **Low strength** | | | | | | | | | |
|---|---|---|---|---|---|---|---|---|---|
| **Example 1** | | | | **Example 3** | | | | | |
| **Initial** | **ACC** | **CRT** | **5°C** | **Initial** | **ACC** | **CRT** | | **5°C** | |
| | **1M** | **1M** | **2M** | | **1M** | **1M** | **3M** | **2M** | **3M** |
| 100.0 % | 92.8 % | 99.4 % | 98.5 % | 100.0 % | 96.6 % | 98.8 % | 99.6 % | 97.7 % | 99.3 % |

**Table 2**

| **High strength** | | | | | | | |
|---|---|---|---|---|---|---|---|
| **Example 2** | | | | | **Example 4** | | |
| **Initial** | **ACC** | **CRT** | | **5°C** | **Initial** | **ACC** | **5°C** |
| | **1M** | **1M** | **3M** | **3M** | | **1M** | **1M** |
| 100.0% | 89.4% | 98.2% | 99.4% | 102.2% | 100.0% | 93.3% | 99.6% |

As evident from Table 1 and 2, the Assay data demonstrates the stability of the pharmaceutical composition of formoterol in the presence of maleic acid. In addition, the data further demonstrate the surprising added benefit of water to form a stable pharmaceutical composition of formoterol.

### Total impurity data (Formoterol fumarate)

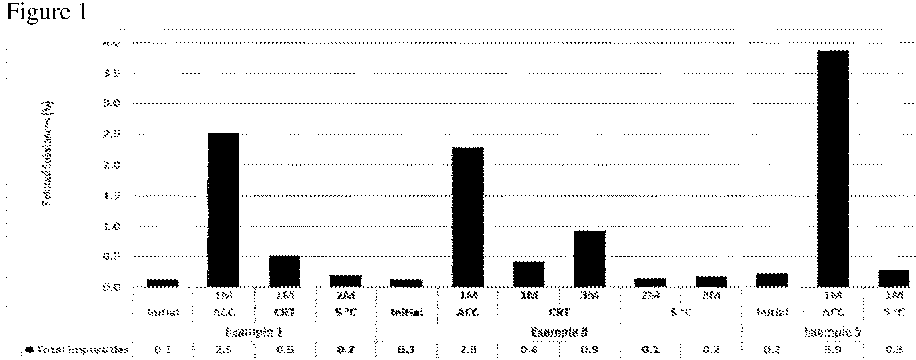

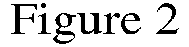

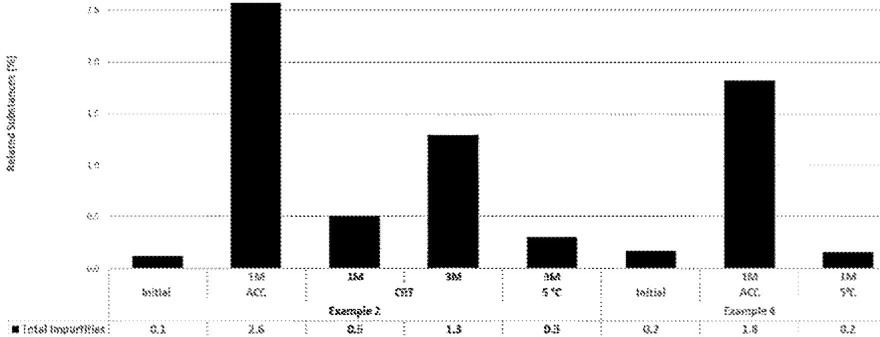

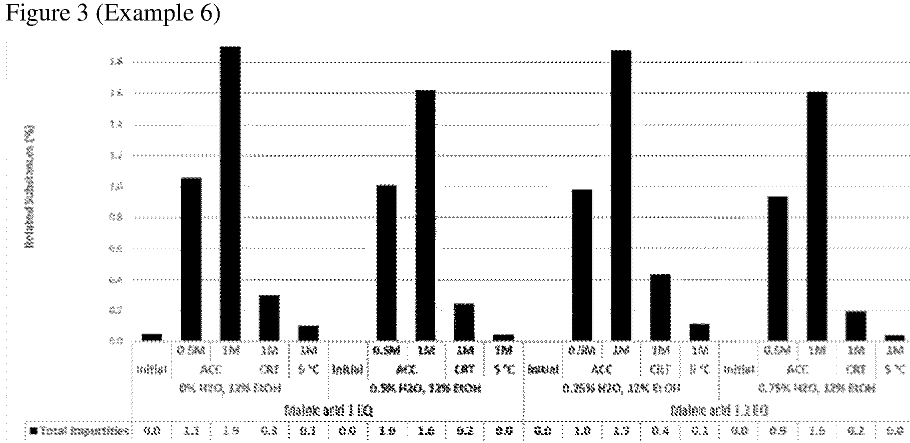

The examples presented above were subjected to various storage conditions to evaluate the chemical stability of these compositions. The accelerated storage condition (ACC) is the harshest, with the samples being subjected to 40 °C and 75% Relative Humidity. Data after 1 month storage at the ACC condition is a good indicator of stability of the composition.

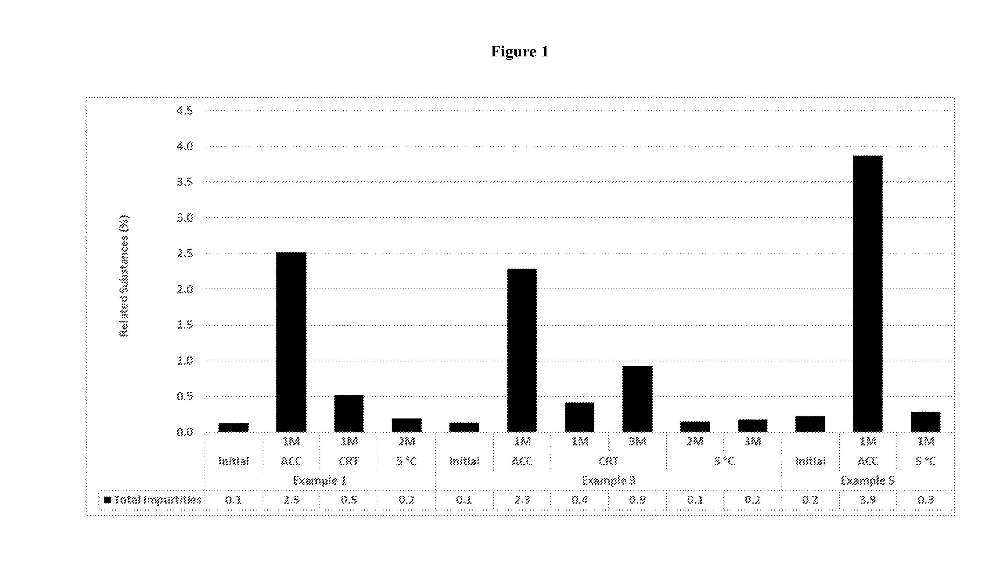

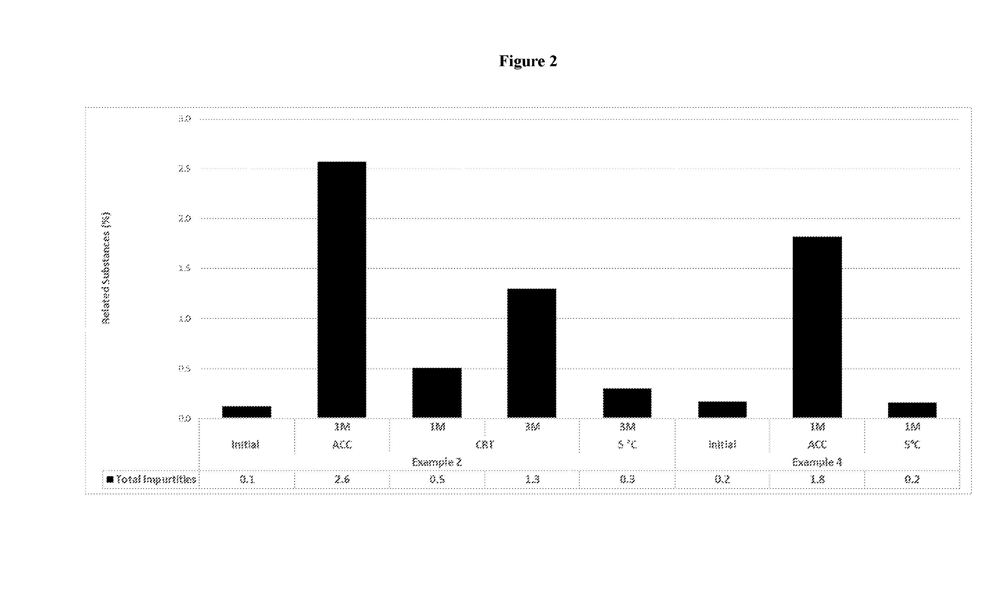

As evident from Figure 1 and 2, the ACC data demonstrates that the composition having a solution of formoterol in the presence of maleic acid was found to be stable in comparison to a composition having a solution of formoterol without organic acid viz., due to significant reduction in the total degradation of the composition with organic acid. Further, the composition, additionally having water, surprisingly demonstrates added benefit in reducing the total degradation of formoterol.

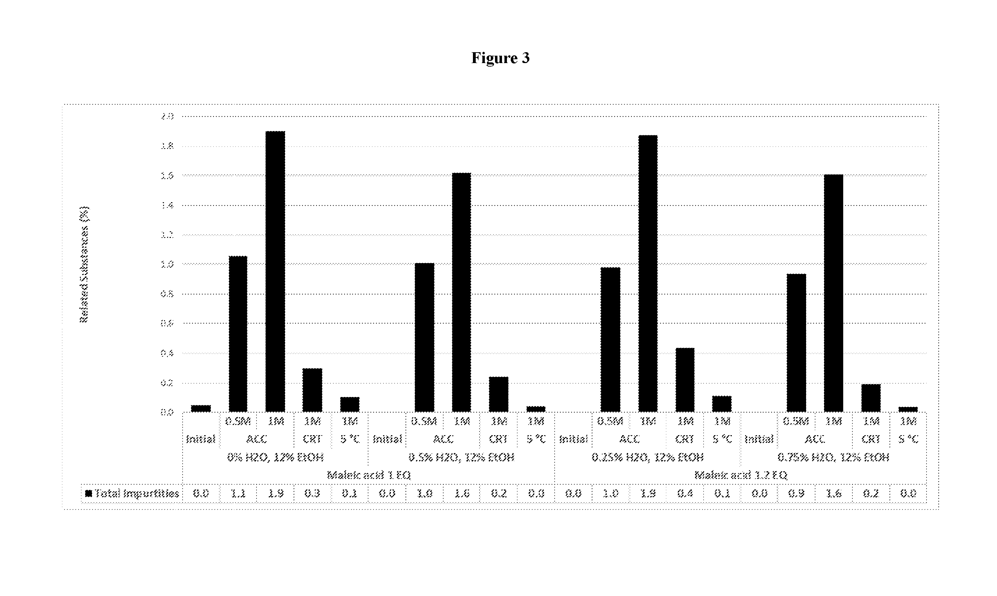

As evident from Figure 3, the ACC data demonstrates that the composition having a solution of formoterol in the presence of maleic acid was found to be stable due to significant reduction in the total degradation of the composition with organic acid.

Further, the composition, additionally having water in varying percentage i.e., 0.5%, 0.75% surprisingly demonstrates added benefit in reducing the total degradation of formoterol.

### Abbreviation

ACC is ICH Accelerated storage condition (40C/75%RH)
CRT is ICH long term storage (25C/60%RH)

## Claims

1. A pharmaceutical composition which is a solution, comprising: a β2-agonist which is formoterol or a pharmaceutically acceptable salt thereof; a corticosteroid which is beclometasone dipropionate; a propellant; a co-solvent; and organic acid; wherein the organic acid is maleic acid.

2. The pharmaceutical composition of claim 1, further comprises water.

3. The pharmaceutical composition of claim 2, wherein the water is present in the amount of up to 3% by weight.

4. The pharmaceutical composition of claim 1, wherein the β2 agonist is present in the composition in an amount from 0.001% to 0.2% by weight.

5. The pharmaceutical composition of claim 1, wherein the propellant is selected from HFA 134a, HFA 227ea and HFA 152a or mixtures thereof.

6. The pharmaceutical composition of claim 1, wherein the co-solvent is selected from a group consisting of dichloromethane, chloroform, ethylacetate, N-methyl pyrrolidone, benzylalcohol, isopropylacetate, acetonitrile, tetrahydrofuran, isopropanol, methanol and ethanol or mixtures thereof.

7. The pharmaceutical composition of claim 1, wherein the co-solvent is present in the composition in an amount from 1% to 40% by weight.

8. The pharmaceutical composition of claim 1, wherein the co-solvent is ethanol.

9. The pharmaceutical composition of claim 8, wherein the co-solvent ethanol is present in concentration of 4 to 20% by weight.

10. The pharmaceutical composition of claim 1, wherein the corticosteroid is present in the composition in an amount from 0.001% to 0.6 % by weight.

11. The pharmaceutical composition of claim 1, is filled in a container having part or all of its internal metallic surfaces made of stainless steel, anodised aluminium or lined with an inert organic coating.

12. The pharmaceutical composition according to any one of the preceding claims for use in the treatment or prophylaxis of a respiratory disorder.

13. The pharmaceutical composition according to claim 12 for use in the treatment or prophylaxis of asthma, COPD, rhinitis or as adjunct therapy for cystic fibrosis, non-cystic fibrosis bronchiectasis, lung infections or pulmonary fibrosis.

## Patentansprüche

1. Pharmazeutische Zusammensetzung in Form einer Lösung, die umfasst: einen β2-Agonisten, der Formoterol oder ein pharmazeutisch annehmbares Salz davon ist; ein Kortikosteroid, das Beclometasondipropionat ist; ein Treibmittel; ein Hilfslösungsmittel; und eine organische Säure; wobei die organische Säure Maleinsäure ist.

2. Pharmazeutische Zusammensetzung nach Anspruch 1, die ferner Wasser umfasst.

3. Pharmazeutische Zusammensetzung nach Anspruch 2, wobei das Wasser in einer Menge von bis zu 3 Gew.-% vorliegt.

4. Pharmazeutische Zusammensetzung nach Anspruch 1, wobei der β2-Agonist in der Zusammensetzung in einer Menge von 0,001 bis 0,2 Gew.-% vorliegt.

5. Pharmazeutische Zusammensetzung nach Anspruch 1, wobei das Treibmittel ausgewählt ist aus HFA 134a, HFA 227ea und HFA 152a oder Gemischen davon.

6. Pharmazeutische Zusammensetzung nach Anspruch 1, wobei das Co-Lösungsmittel ausgewählt ist aus einer Gruppe bestehend aus Dichlormethan, Chloroform, Ethylacetat, N-Methylpyrrolidon, Benzylalkohol, Isopropylacetat, Acetonitril, Tetrahydrofuran, Isopropanol, Methanol und Ethanol oder Gemischen davon.

7. Pharmazeutische Zusammensetzung nach Anspruch 1, wobei das Co-Lösungsmittel in der Zusammensetzung in einer Menge von 1 bis 40 Gew.-% vorliegt.

8. Pharmazeutische Zusammensetzung nach Anspruch 1, wobei das Co-Lösungsmittel Ethanol ist.

9. Pharmazeutische Zusammensetzung nach Anspruch 8, wobei das Co-Lösungsmittel Ethanol in einer Konzentration von 4 bis 20 Gew.-% vorliegt.

10. Pharmazeutische Zusammensetzung nach Anspruch 1, wobei das Kortikosteroid in der Zusammensetzung in einer Menge von 0,001 bis 0,6 Gew.-% vorliegt.

11. Pharmazeutische Zusammensetzung nach Anspruch 1, die in einen Behälter enthalten ist, dessen innere Metalloberflächen ganz oder teilweise aus Edelstahl, eloxiertem Aluminium bestehen oder mit einer inerten organischen Beschichtung ausgekleidet sind.

12. Pharmazeutische Zusammensetzung nach einem der vorhergehenden Ansprüche zur Verwendung bei der Behandlung oder Prophylaxe einer Atemwegserkrankung.

13. Pharmazeutische Zusammensetzung nach Anspruch 12 zur Verwendung bei der Behandlung oder Prophylaxe von Asthma, COPD, Rhinitis oder als Begleittherapie bei zystischer Fibrose, nicht-zystischer Bronchiektasie, Lungeninfektionen oder Lungenfibrose.

## Revendications

1. Composition pharmaceutique qui est une solution, comprenant : un agoniste β2 qui est formotérol ou un sel pharmaceutiquement acceptable de celui-ci ; un corticostéroïde qui est du dipropionate de béclométasone ; un gaz propulseur ; un co-solvant ; et un acide organique ; dans laquelle l'acide organique est de l'acide maléïque.

2. Composition pharmaceutique de la revendication 1, comprenant en outre de l'eau.

3. Composition pharmaceutique de la revendication 2, dans laquelle l'eau est présente en la quantité de 3 % en poids au maximum.

4. Composition pharmaceutique de la revendication 1, dans laquelle l'agoniste β2 est présent dans la composition en une quantité de 0,001 % à 0,2 % en poids.

5. Composition pharmaceutique de la revendication 1, dans laquelle le gaz propulseur est sélectionné parmi HFA 134a, HFA 227ea, et HFA 152a, ou des mélanges de ceux-ci.

6. Composition pharmaceutique de la revendication 1, dans laquelle le co-solvant est sélectionné parmi un groupe constitué de : dichlorométhane, chloroforme, éthylacétate, pyrrolidone N-méthylique, alcool benzylique, acétate d'isopropyle, acétonitrile, tétrahydrofurane, isopropanol, méthanol, et éthanol ou des mélanges de ceux-ci.

7. Composition pharmaceutique de la revendication 1, dans laquelle le co-solvant est présent dans la composition en une quantité de 1 % à 40 % en poids.

8. Composition pharmaceutique de la revendication 1, dans laquelle le co-solvant est de l'éthanol.

9. Composition pharmaceutique de la revendication 8, dans laquelle le co-solvant éthanol est présent en une concentration de 4 à 20 % en poids.

10. Composition pharmaceutique de la revendication 1, dans laquelle le corticostéroïde est présent dans la composition en une quantité de 0,001 % à 0,6 % en poids.

11. Composition pharmaceutique de la revendication 1, en laquelle est rempli un contenant ayant une partie ou la totalité de ses surfaces métalliques internes faite d'acier inoxydable, d'aluminium anodisé, ou enduite d'un revêtement organique inerte.

12. Composition pharmaceutique selon l'une quelconque des revendications précédentes pour utilisation dans le traitement ou la prophylaxie d'un trouble respiratoire.

13. Composition pharmaceutique selon la revendication 12 pour utilisation dans le traitement ou la prophylaxie de : asthme, COPD, rhinite, ou en tant que thérapie adjuvante pour la fibrose cystique, la fibrose non cystique, la bronchectasie, des infections des poumons, ou la fibrose pulmonaire.
